(19) Europäisches Patentamt
European Patent Office
Office européen des brevets

(11) **EP 4 261 837 A1**

(12) **EUROPEAN PATENT APPLICATION**

(43) Date of publication:
**18.10.2023 Bulletin 2023/42**

(21) Application number: **22168347.7**

(22) Date of filing: **14.04.2022**

(51) International Patent Classification (IPC):
**G16H 20/30** (2018.01)

(52) Cooperative Patent Classification (CPC):
**G16H 20/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Polar Electro Oy**
**90440 Kempele (FI)**

(72) Inventors:
- **Peltonen, Jussi**
  **90440 Kempele (FI)**
- **Granlund, Tommy**
  **90440 Kempele (FI)**

(74) Representative: **Kolster Oy Ab**
**Salmisaarenaukio 1**
**P.O. Box 204**
**00181 Helsinki (FI)**

(54) **APPARATUS, METHOD, AND COMPUTER PROGRAM PRODUCT FOR PROVIDING TRAINING GUIDANCE**

(57) An apparatus, method and computer program product for providing training guidance. The method comprises: acquiring (200) time series data that represents power output of athletic performances of a user (100); extracting (202) a plurality of segments from the time series data; determining (204) segment power values on the basis of the time series data of the extracted plurality of segments; forming (206) a set of scatter points describing the segment power values as a function of respective durations of the extracted plurality of segments, and fitting (208) a performance power curve for the set of scatter points; determining (210) a set of training zones on the basis of the performance power curve; generating (212) a training instruction on the basis of the set of training zones; and causing (214) a user interface to output the training instruction to the user.

FIG. 1

EP 4 261 837 A1

**Description**

FIELD

Various embodiments relate to an apparatus, method, and computer program product for providing training guidance.

BACKGROUND

Sports performance testing is traditionally based on laboratory testing of athletes. However, laboratory testing is time-consuming and expensive, and the results may not translate well to real-world performance outside the laboratory.

A mathematical model that describes running performance has been recently studied in Mulligan et al. (2018), A minimal power model for human running performance, PLoS ONE 13(11): e0206645. The model may utilize real-world data, such as world record times, in contrast to data collected in a laboratory setting. Further study of the model has been performed in Emig & Peltonen, Human running performance from real-world big data, Nat Commun 11, 4936 (2020). However, practical applications of the model are currently very limited, and further development is needed.

BRIEF DESCRIPTION

According to an aspect, there is provided subject matter of independent claims. Dependent claims define some embodiments.

One or more examples of implementations are set forth in more detail in the accompanying drawings and the description of embodiments.

LIST OF DRAWINGS

Some embodiments will now be described with reference to the accompanying drawings, in which

FIG. 1 illustrates embodiments of an apparatus for providing training guidance;
FIG. 2 illustrates embodiments of a method for providing training guidance;
FIG. 3 and FIG. 4 illustrate embodiments of a performance power curve;
FIG. 5 and FIG. 6 illustrate embodiments related to providing training guidance;
FIG. 7 and FIG. 8 illustrate embodiments related to fitting the performance power curve;
FIG. 9 and FIG. 10 illustrate embodiments related to acquisition of time series data;
FIG. 11 illustrates embodiments for using and updating the performance power curve; and
FIG. 12 illustrates embodiments of an apparatus for providing training guidance as a block diagram.

DESCRIPTION OF EMBODIMENTS

The following embodiments are only examples. Although the specification may refer to "an" embodiment in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

Reference numbers, both in the description of the embodiments and in the claims, serve to illustrate the embodiments with reference to the drawings, without limiting it to these examples only.

The embodiments and features, if any, disclosed in the following description that do not fall under the scope of the independent claims are to be interpreted as examples useful for understanding various embodiments of the invention.

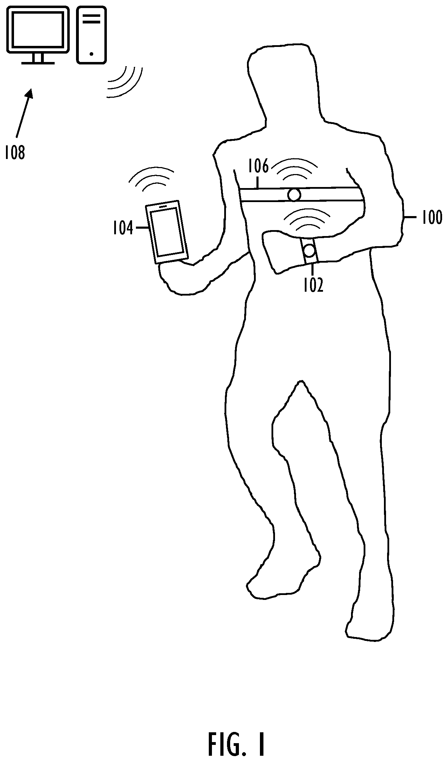

FIG. 1 illustrates embodiments of an apparatus for providing training guidance to a user 100. The user 100 is illustrated to be performing a physical exercise or an athletic performance. The physical exercise may be running (such as in FIG. 1), cycling, swimming, cross-country skiing, rowing, or any other sport for which power output may be directly or indirectly measured. The power output may refer to power generated or output by the user during the athletic performance.

The apparatus of FIG. 1 may be or comprise a wearable device 102, a portable electronic device 104, a wearable sensor device 106, and/or a server computer 108. The apparatus may be a single physical apparatus or a distributed apparatus (a system). For example, the wearable device 102 may operate as the single physical apparatus. The distributed apparatus or system may comprise a plurality of communicatively coupled physical devices such as the wearable device 102, the wearable sensor device 106, the portable electronic device 104, and/or the server computer 108.

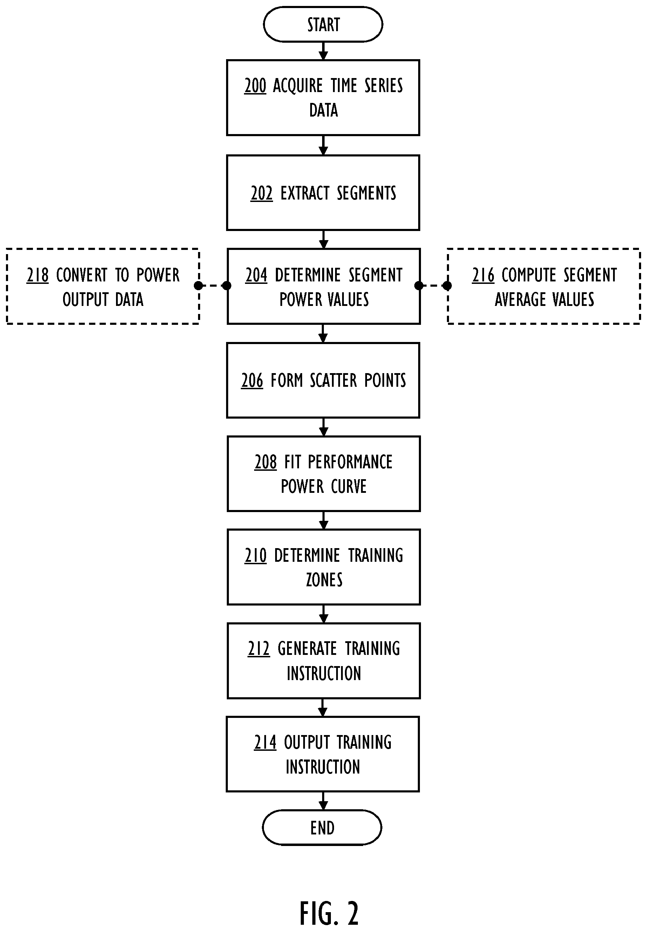

The apparatus may be configured to perform a method illustrated in FIG. 2. The method of comprises: acquiring 200 time series data that represents power output of athletic performances by the user; extracting 202 a plurality of segments from the time series data, wherein the plurality of segments comprise a first segment having a first duration and a second segment having a second duration, the second duration being different from the first duration; determining 204 segment power values on the basis of the time series data of the extracted plurality of segments; forming 206 a set of scatter points describing the segment power values as a function of respective durations of the extracted plurality of segments, and fitting 208 a performance power curve for the set of scatter points; determining 210 a set of training zones on the basis of the performance power curve; generating 212 a training instruction on the basis of the set of training zones; and causing 214 a user interface to output the training instruction.

The solutions described herein provide individualized training guidance to the user. The performance power curve and the training zones derived from it characterize the user's athletic performance. By generating the training instruction on the basis of the set of training zones, the user may receive individualized feedback and guidance on their training.

The time series data represents power output of athletic performances performed by the user. Power output may be directly measured by power sensors, commonly used with (stationary) bicycles, for example. In an embodiment, the time series data is power data measured by a power sensor. Power output may also be derived from other kinds of time series data. It has been found that linear relationships exist between power, velocity, and heart rate. These linear relationships may be exploited to convert e.g. velocity data and/or heart rate data to power data, or vice versa. Further, the apparatus may derive velocity data from e.g. location data measured by a location sensor, and/or the apparatus may derive heart rate data from electrocardiogram (ECG) data measured by an ECG sensor, for example.

The time series data may be time series data of past athletic performances that is acquired from one or more memories, and/or time series data acquired in real time by the apparatus. The apparatus may comprise or be coupled to one or more sensors configured to measure the time series data, and the apparatus may be configured to acquire the time series data from the one or more sensors. The acquiring may also comprise receiving, over a wired or wireless connection, the time series data from one or more sensors or one or more memories.

The apparatus is configured to extract 202 a plurality of segments from the time series data. The plurality of segments comprise a first segment having a first duration and a second segment having a second duration, and the second duration is different from the first duration. In principle, at least two different durations may be required to fit the performance power curve. Alternatively or additionally, durations of the segments may be examined via distances travelled during the segments. For example, when the time series data comprises location data, the apparatus may determine a distance travelled by the user on the basis of the location data. The apparatus may select the segments to be extracted on the basis of an assumption that different distances are generally travelled in different durations of time. The first segment may thus have a first distance and the second segment may have a second distance, and the second distance is different from the first distance.

The apparatus is configured to determine 204 segment power values on the basis of the time series data of the extracted plurality of segments. In an embodiment, the apparatus is configured to compute 216 segment average values from the time series data of the extracted plurality of segments to determine the segment power values. The segment power values may therefore be segment average power values. The apparatus may compute an average value for each segment from the time series data of the respective segment. Average values have been observed to sufficiently characterize the user's performance, and are computationally efficient compared to some other statistical parameters, for example.

In an embodiment, the time series data comprises velocity data and/or heart rate data, and the apparatus is configured to convert 218 the velocity data and/or the heart rate data to power output data, and to determine the segment power values on the basis of the power output data. When the apparatus is configured to compute segment average values, the average values may be computed before or after then conversion to power output data.

When the time series data comprises heart rate data, the apparatus may perform a heart rate - power conversion on the heart rate data to obtain the power output data. The heart rate - power conversion may be a transformation of the form $P = A(HR) + b$, wherein $P$ represents power, $HR$ represents heart rate, $A$ represents a linear mapping coefficient, and $b$ represents a translation parameter. The translation parameter $b$ may be zero.

Heart rate is linearly proportional to power at least under certain conditions. The heart rate of the user and power output by the user are linearly proportional at least when the user is performing aerobic exercise, in contrast with anaerobic exercise. The phenomenon of heart rate drift may also distort the relationship between heart rate and power. Heart rate drift is observed when heart rate increases despite a constant power output. A rapid change in exercise intensity may result in a large heart rate drift. A decrease in blood volume, an increase in core temperature, and neuromuscular fatigue may result in a gradual heart rate drift. The apparatus may be configured to detect, on the basis of the time series data, heart rate drift, or if the user is performing anaerobic exercise. If neither heart rate drift is detected nor is the user performing anaerobic exercise, the apparatus may perform the heart rate-power conversion on the heart rate data to obtain the power output data.

When the time series data comprises velocity data, the apparatus may perform a velocity-power conversion on the velocity data to obtain the power output data. The velocity-power conversion may be a transformation of the form P = Cv + d, wherein P represents power, v represents velocity, C represents a linear mapping coefficient, and d represents a translation parameter. The translation parameter d may be zero.

Velocity is directly proportional to power in some sports such as running, but not in others such as cycling. The apparatus may be configured to detect, on the basis of the time series data, the sport of the athletic performance performed by the user. Alternatively or additionally, the apparatus may receive the sport from the user as an input via the user interface. The apparatus may compare the detected sport to a list of sports stored e.g. in a memory of the apparatus, for which sports velocity is proportional to power. If the sport is included in the list, the apparatus may perform the velocity-power conversion on the velocity data to obtain the power output data.

The apparatus may utilize multimodal conversions, such as Running Power by Polar, to obtain the power output data. The apparatus may compute Running Power from velocity and gradient time series data obtained from global positioning system (GPS) and barometric sensors, resulting in power output data. The apparatus may determine the segment power values on the basis of the power output data.

The apparatus is configured to form 206 a set of scatter points describing the segment power values as a function of respective durations of the extracted plurality of segments. The apparatus may obtain durations of the extracted plurality of segments from timestamps or metadata of the time series data, for example. The apparatus is further configured to fit 208 a performance power curve for the set of scatter points.

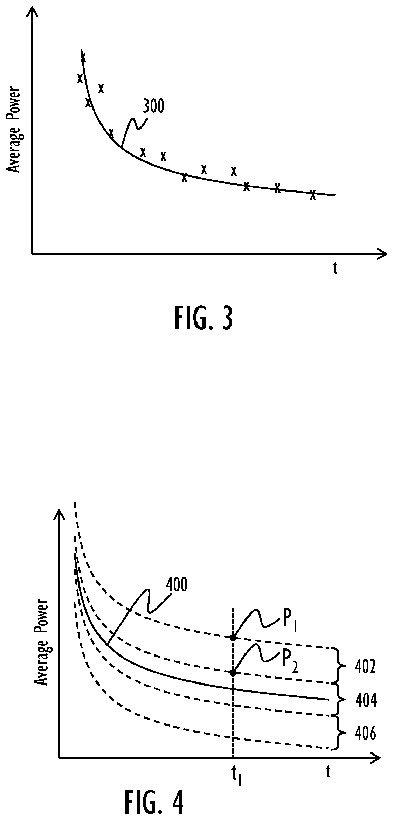

FIG. 3 illustrates embodiments of the set of scatter points and the performance power curve 300. In FIG. 3, the scatter points are plotted in a coordinate system wherein the vertical axis represents average power, and the horizontal axis represents duration. In FIG. 3, the segment power values are segment average power values. The performance power curve 300 is fitted to the set of scatter points using e.g. the method of least squares.

As described above, velocity or speed may be directly proportional to power in some conditions. The following paragraphs describe a mathematical model, also known as a universal performance model, in terms of speed. The model expresses exercise intensity on a relative power scale, which varies between zero corresponding to basal metabolic rate, and unity at maximal aerobic power (MAP) generation. Alternatively or additionally, the model may be represented in terms of power or heart rate, with the help of e.g. conversions similar to those described above.

The relation between a fastest performance time $T$ and distance $d$ is characterized by two equations (1) and (2) shown below. Equation (1) is applicable when $d >= d_c$, and equation (2) is applicable when $d <= d_c$, wherein $d_c$ is a crossover distance given by $d_c = t_c v_c$, wherein $t_c$ and $v_c$ are crossover time and speed. The crossover speed corresponds to speed or velocity at maximal oxygen consumption (VO2max), and crossover distance is a distance that is travelled in time $t_c$ with speed $v_c$. Parameter $v_c$ coincides with a speed at which VO2max can be achieved and therefore it is also known as maximal aerobic speed (MAS). It has been estimated that $t_c$ may be 5 to 7 minutes. In an embodiment, $t_c$ is 6 minutes.

$$T(d) = \frac{t_c}{\gamma_l}\frac{d}{d_c}\frac{1}{W_{-1}\left[\frac{d}{d_c}\frac{exp(-1/\gamma_l)}{\gamma_l}\right]} \qquad (1)$$

$$T(d) = \frac{t_c}{\gamma_s}\frac{d}{d_c}\frac{1}{W_{-1}\left[\frac{d}{d_c}\frac{exp(-1/\gamma_s)}{\gamma_s}\right]} \qquad (2)$$

Parameters $\gamma_s$ and $\gamma_l$ are related to endurance parameters $E_s$ and $E_l$. Long distance endurance parameter $E_l$ is given by the relation $E_l = exp(0.1/\gamma_l)$, and short distance endurance parameter $E_s$ is given by the relation $E_s = exp(0.1/\gamma_s)$. The subscripts $l$ and $s$ refer to long and short distance performance, respectively. $W_{-1}$ is real branch of the Lambert W-function, which is defined as the multi-valued inverse of the function $w \to w\,exp(w)$.

$E_l$ is also known as Endurance index, i.e. a ratio of a duration for which an athlete is able to maintain 90% of MAS and a duration for which the athlete is able to maintain MAS. For example, if an athlete can maintain 90% of MAS for 18 minutes, and MAS for 6 minutes, the athlete's endurance index is 18/6 = 3.

The model described above may be utilized to acquire a threshold power curve. In an embodiment, the performance power curve is the threshold power curve. The performance power curve may be a power law or a broken power law. Even though the performance power curve is described herein in the form of a power vs. duration function, power and/or duration may be replaced by other parameters whose relationship with power is known or may be estimated. For example, power may be replaced by heart rate or velocity, and/or duration may be replaced by distance. The apparatus may determine the relationship between duration and distance using velocity data and a formula $d = tv$, wherein $d$ is distance, $t$ is duration, and $v$ is velocity. Further, the variables power and duration (and/or their replacements) may be switched around so that the scatter points describe the durations of the extracted plurality of segments as a function of segment power values, for example.

Training zones have been previously based on the idea of "threshold power" that describes power that muscles can supply for unlimited amount of time. This time-invariant threshold power has led to static training zones that are also time-invariant. However, this defies observations as no power can be sustained without limitations. Analysis of running world records have revealed that maximal power that runner can sustain decreases rapidly over first 12 minutes and then continues to drop indefinitely at slower rate.

The apparatus is configured to determine 210 a set of training zones on the basis of the performance power curve. Determining the set of training zones on the basis of the performance power curve may refer to determining the set of training zones on the basis the curve itself (e.g. the shape and/or values of the curve), and/or on the basis of one or more parameters characterizing the curve. FIG. 4 illustrates a performance power curve 400 in an average power vs. duration coordinate system. Each training zone may define an (average) power range for each duration t. In FIG. 4, three training zones 402, 404, 406 have been determined on the basis of the performance power curve. Training zone 402 includes power values above the performance power curve, training zone 404 includes power values equal to, above, and below the performance power curve, and training zone 406 includes power values below the performance power curve. The training zones may be non-overlapping as illustrated in FIG. 4.

In an embodiment, the apparatus is configured to determine a training zone 402, 404, 406 of the set of training zones by multiplying the performance power curve 400 by a constant multiplier. This way, information on the relationship between power and duration contained in the performance power curve may be efficiently transferred to the set of training zones, and the training zones may closely follow the shape of the performance power curve. The apparatus may generate a boundary for the training zone by multiplying the performance power curve 400 by the constant multiplier. In an embodiment, each training zone 402, 404, 406 of the set of training zones is defined by the performance power curve 400 multiplied by a constant multiplier. The constant multiplier for each training zone may be different, i.e. zone-specific.

As an example of some of the above embodiments, three training zones may be defined as follows. A first training zone, a "recovery" training zone, may be defined with the performance power curve multiplied by 0.95 as an upper bound. A second training zone, a "productive" training zone, may be defined with the performance power curve multiplied by 0.95 as a lower bound, and the performance power curve multiplied by 1.05 as an upper bound. A third training zone, a "maximal" training zone, may be defined with the performance power curve multiplied by 1.05 as a lower bound. The training zones are not necessarily completely bounded; the first training zone may not have a lower bound, and/or the third or last training zone may not have an upper bound.

In an embodiment, the constant multiplier is between 0 and 1. As an example, three training zones may be defined in the order of increasing power as follows: A first training zone, a "recovery" training zone, may be defined with the performance power curve multiplied by 0.5 as a lower bound and the performance power curve multiplied by 0.7 as an upper bound. A second training zone, a "productive" training zone, may be defined with the performance power curve multiplied by 0.7 as a lower bound and the performance power curve multiplied by 0.9 as an upper bound. A third training zone, a "maximal" training zone, may be defined with the performance power curve multiplied by 0.9 as a lower bound.

In an embodiment, the training zones are completely bounded (in terms of power). Considering again the above example, the third training zone may have an upper bound of the performance power curve multiplied by 1.0 or even 1.1, resulting in three completely bounded training zones.

It is also noted that the number of training zones in the set of training zones is not limited to three but may be any suitable number, such as 2, 3, 4, or 5, for example.

The apparatus is configured to generate 212 a training instruction on the basis of the set of training zones. The training instruction may guide the user regarding which training zone is suitable for which duration, and what zone or power to select for lighter or heavier training sessions. The apparatus is configured to cause 214 a user interface to output the training instruction to the user 100.

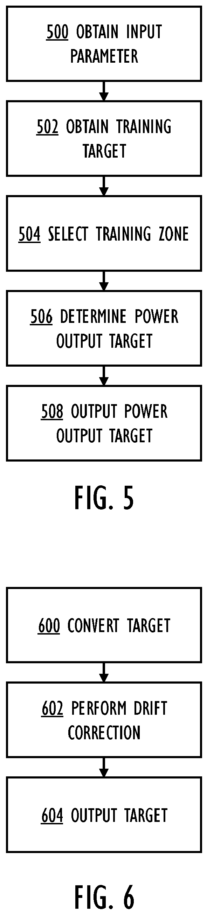

FIG. 5 illustrates an embodiment comprising obtaining 500, 502 an input parameter and a training target; selecting 504, on the basis of the training target, a training zone from the set of training zones; determining 506 a power output target on the basis of the input parameter and the selected training zone; and causing 508 the user interface to output the power output target. In an embodiment, the input parameter is a duration parameter or a distance parameter. The training target may be a qualitative target such as "recovery", "development", or "race" describing a goal of a training session or part of the training session, for example. The apparatus selects a training zone for the training target from the set of training zones. A mapping of training targets and training zones may be stored in a memory of the apparatus, e.g. the target "recovery" may correspond to a recovery training zone, a "development" target may correspond to a productive training zone, and a "race" target may correspond to a maximal training zone. The apparatus may be configured to select the training zone using the mapping. The apparatus selects a power output target on the basis of the input parameter and the selected training zone. FIG. 4 illustrates an example wherein the input parameter is a duration parameter ti, the training target is "race", and the selected training zone is maximal training zone 402. The apparatus may determine power values $P_1$ and $P_2$ that correspond to the upper and lower bounds of the training zone at duration $t_1$. The apparatus may determine the power output target as a range, such as $[P_1, P_2]$, or as a power value, such as the average of $P_1$ and $P_2$. The apparatus causes the user interface to output the power output target to the user as e.g. a value or a range of values, in Watts, for example.

The apparatus may obtain the input parameter and/or the training target from the user as a user input via the user interface. Alternatively or additionally, the apparatus may read the input parameter and/or the training target from a memory of the apparatus, and/or the apparatus may receive the input parameter and/or the training target over a wireless connection from another apparatus, e.g. a server computer.

FIG. 6 illustrates embodiments related to the power output target. In an embodiment, the apparatus is configured to convert 600 the power output target to a heart rate target and/or a velocity target, and to cause 604 the user interface to output the heart rate target and/or the velocity target as the power output target. Power may be difficult to understand especially by inexperienced users, and conversion to heart rate or velocity may make the target better understandable to the users. Further, power data may be more difficult to acquire than velocity or heart rate data, and comparison of the target to acquired velocity and/or heart rate data is thus facilitated. The velocity-power conversion and/or the heart rate - power conversion described earlier, or another conversion, may be utilized to convert the power output target to a heart rate target and/or a velocity target.

In an embodiment, the apparatus is configured to convert the power output target to a heart rate target and to perform 602 a drift correction on the heart rate target, and to cause the user interface to output the corrected heart rate target as the power output target. Heart rate drift, as described earlier, corresponds to an increase in heart rate despite constant power output. When the power output target is output as a heart rate target, the heart rate target may be incorrect or even unachievable if the user is experiencing heart rate drift. The heart rate target may thus be corrected using a drift correction to adjust, typically increase, the heart rate target to one that more accurately represents the desired power output target. The drift correction may be based on heart rate demand, which represents a true heart rate that user's effort requires. When the relation between heart rate and heart rate demand is known, the user may be instructed to achieve a suitable heart rate even in the case of heart rate drift. A model describing the relation between heart rate and heart rate demand is described in Stirling, James Robert, et al., A Model of Heart Rate Kinetics in Response to Exercise. Journal of Nonlinear Mathematical Physics, October 2008, Volume 15, Supplement 3, pages 426-436.

The apparatus may be further configured to acquire power data, velocity data, and/or heart rate data of the user. The acquiring may be performed similarly to that of the time series data. The apparatus may be configured to cause the user interface to output the acquired power data, velocity data, and/or heart rate data. The apparatus may be configured to compare the power data to the power output target, to compare the velocity data to the velocity target, and/or to compare the heart rate data to the heart rate target. If the power/velocity/heart rate data does not meet or fulfil the corresponding target, the apparatus may be configured to cause the user interface to output an indication to the user. Meeting the target herein may refer to the data falling within the target range, for example. The indication may be a graphical indication displayed on a display screen or another display unit, an audio indication output by an audio output device such as a loudspeaker, or a haptic indication output by a haptic output device configured to provide haptic indications to a user, for example.

The performance power curve may characterize both aerobic and anaerobic performance of the user. In order to acquire an accurate estimate of the aerobic and anaerobic performance of the user, the time series data preferably includes data from both kinds of athletic performances. In an embodiment, the first duration is smaller than a crossover time representing a time for maintaining maximal aerobic speed (MAS), and the second duration is greater than the crossover time. As described earlier, the crossover time $t_c$ may be 5 to 7 minutes, or in an embodiment, $t_c$ is 6 minutes.

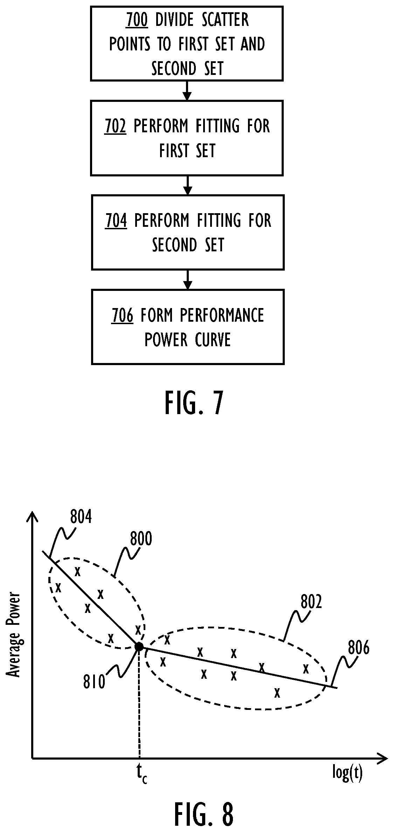

In an embodiment illustrated in FIG. 7 and FIG. 8, the apparatus is configured to divide 700 the set of scatter points to a first set of scatter points 800 and a second set of scatter points 802, wherein durations of the first set of scatter points are smaller than or equal to a cut-off time, and durations of the second set of scatter points are greater than or equal to the cut-off time; and perform 702 the fitting for the first set of scatter points to obtain a first piece 804 of the performance power curve, and perform 704 the fitting for the second set of scatter points to obtain a second piece 806 of the performance power curve, and form 706 the performance power curve on the basis of the first piece of the performance power curve and the second piece of the performance power curve. It has been observed that the performance power curve may be best fit in 2 pieces. The performance power curve may thus be a (2-piece) piecewise function. In an embodiment, the cut-off time is the crossover time ($t_c$). Optimal fitting may be achieved when the performance power curve is a piecewise function with a breakpoint 810 at $t_c$. The apparatus may perform the fitting such that the breakpoint is fixed at the crossover time. Such a fitting separates aerobic and anaerobic performances to the first and second pieces of the performance power curve, respectively, and allows for more accurate characterization of the user's performance as individuals may have drastically different aerobic and anaerobic performance characteristics. Athletic performances with a duration smaller than or equal to $t_c$ may be predominantly anaerobic or at least representative of anaerobic performances, and athletic performances with a duration greater than or equal to $t_c$ may be predominantly aerobic or at least representative of aerobic performances.

In FIG. 8 the duration is expressed on a logarithmic scale. This facilitates advantages regarding the fitting and the form of the performance power curve. In an embodiment, the performance power curve is a piecewise linear function. Fitting of linear functions is computationally simple and possibly more accurate that fitting more complex functions. The performance profile curve may be defined by parameters such as the respective slopes $E, F,$ of pieces 804 and 806 and the breakpoint 810. The breakpoint may correspond to duration $t_c$.

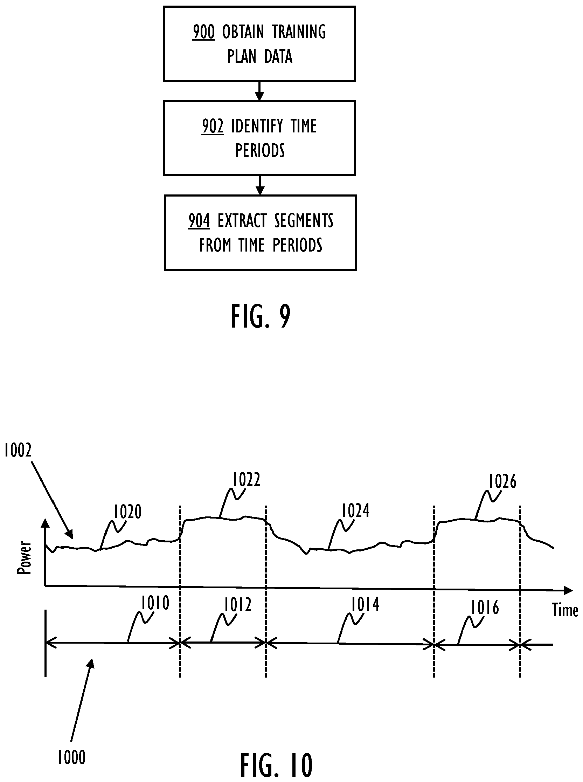

FIG. 9 and FIG. 10 illustrate embodiments related to acquisition of the time series data. In an embodiment, the apparatus is configured to obtain 900 training plan data 1000 associated with the time series data 1002; identify 902, on the basis of the training plan data, one or more time periods of the time series data that have training intensities greater than an inclusion threshold; and extract 904 the plurality of segments from the time series data of the identified one or more time periods. The training plan data may comprise training program and/or training session plan data. The training program may comprise a long-term plan that spans over several days, weeks or months. For example, a training program that follows a 3 week cycle may comprise the following entries: 1) Productive week (2 key exercises), 2) Productive week (1 key exercise), and 3) Recovery week. Key exercises may refer to high-intensity exercises that are expected to produce time series data that is suitable for the purposes described herein. The apparatus may be configured to identify the one or more time periods as one or more key exercises on the basis of the training plan data. The training intensities may be defined as number of training sessions per week, energy expenditure or calories burned, total or average power, total duration, or other types of data that may be measured by sensors described further in this document. The training intensities may refer to planned or measured training intensities. The inclusion threshold may be a value of one or more of the aforementioned types of data. The apparatus may or may not acquire the time series data of the entire one or more time periods; the apparatus may be configured to acquire (only) a part of the time series data of the identified one or more time periods.

The training session plan data may comprise information on training intervals and other time periods within a single training session. The training plan data of FIG. 10 illustrates intervals 1010,1012,1014 and 1016. The time series data segments corresponding to these intervals are 1020, 1022, 1024, 1026, respectively. Of these training interval periods, 1010 and 1014 may be low-intensity training interval periods, and 1012 and 1016 may be high-intensity interval periods. Information on the intensity of the training interval periods may be encoded within the training plan data. In an embodiment, the one or more time periods comprise one or more training interval periods 1012, 1016, and the apparatus is configured to extract one or more of the plurality of segments as the one or more training interval periods. The apparatus may thus extract time series data segments 1022 and 1026 corresponding to training interval periods 1012, 1016.

In an embodiment, the apparatus is configured to acquire time series data that represents power output of athletic performances of one or more users. The one or more users may include or exclude the user 100. When the one or more users does not include the user 100, the apparatus may provide the training instruction to the user 100 without having to acquire time series data from athletic performances of the user 100. The user therefore does not necessarily need to perform any athletic performances for the data acquisition, but still receives the training instruction from the apparatus.

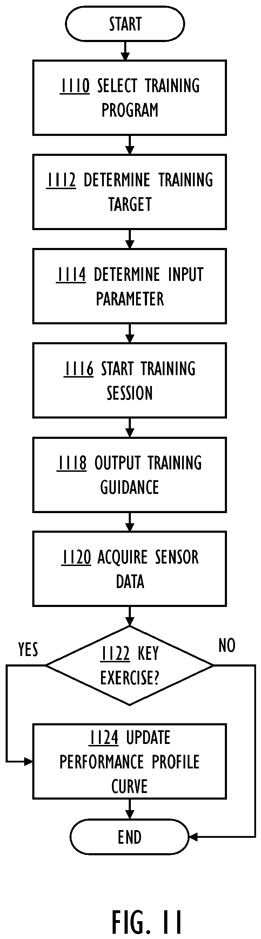

FIG. 11 illustrates embodiments related to utilizing and updating the performance power curve during a training session. The apparatus may be configured to select 1110 a training program e.g. in response to a user input. The user may indicate that they wish to train e.g. for a marathon, and provide a user input to the apparatus selecting a corresponding training program e.g. from a selection of training programs displayed on the user interface. The apparatus may be configured to determine 1112, 1114 the training target and the input parameter e.g. on the basis of the training program by selecting the training target and duration and/or distance of a scheduled training session, for example. The apparatus may be configured to start 1116 the training session with the training target and input parameter specified above. During the training session, the apparatus may cause 1118 the user interface to output training guidance based on the set of training zones to the user. The apparatus may be configured to acquire 1120 sensor data or further time series data during the training session. The apparatus may determine 1122 whether the training session is/was a key exercise, and if so, the apparatus may be configured to extract one or more segments from the sensor data or further time series data acquired during the training session. The apparatus may be configured to update 1124 the performance profile curve and the set training zones on the basis of the sensor data, or specifically on the basis of the segments extracted from the sensor data. The updating may be performed as in steps 204, 206, 208 and/or 210 of the method of FIG. 2, but using the segments extracted from the time series data acquired earlier and the sensor data acquired during the training session.

One or more of the steps of the above embodiments may be combined with the method of FIG. 2 and/or its embodiments to provide a self-updating training guidance solution.

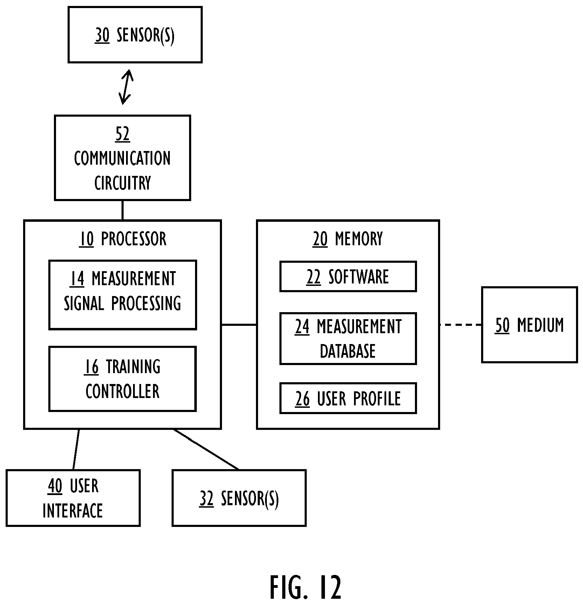

FIG. 12 illustrates embodiments of an apparatus or part of a system as a block diagram. The apparatus may be the wearable device 102, the portable electronic device 104, the wearable sensor device 106, or the server computer 108, for example. The apparatus of FIG. 12 may comprise a processing system configured to perform the method of FIG. 2, or any one of the embodiments thereof. The processing system may comprise at least one processor 10 and at least one memory 20. The apparatus may further comprise a user interface 40 comprising a display screen or another display unit, an input device such as one or more buttons and/or a touch-sensitive surface, and an audio output device such as a loudspeaker. In some embodiments, the user interface 40 comprises a haptic output device configured to provide haptic indications to a user.

The processor 10 may comprise a measurement signal processing circuitry 14 configured to process the time series data by controlling at least some of the steps of the procedure of FIG. 2, such as steps 200, 202, 204, 206, and 208, or any one of the embodiments thereof. The measurement signal processing circuitry 14 may be configured to acquire time series data that represents power output of athletic performances from sensors 30 and/or from sensors 32 connected to the processor 10. The processor 10 may further comprise a training controller (circuitry) 16 configured to control training-related steps of the procedure of FIG. 2, such as generating 212 and outputting 214 the training instruction. The measurement signal processing circuitry 14 or the training controller 16 may be configured to determine 210 the training zones, depending on the implementation.

The apparatus may comprise a communication circuitry 52 connected to the processor 10. The communication circuitry 52 may comprise hardware and software suitable for supporting e.g. Bluetooth® communication protocol such as Bluetooth Smart specifications. It should be appreciated that other communication protocols are equivalent solutions as long as they are suitable for a personal area network (PAN) or suitable for measurement scenarios described in this document. The communication circuitry may comprise a radio modem and appropriate radio circuitries for establishing a communication connection between a server computer and a wearable device. Suitable radio protocols may include IEEE 802.11-based protocols or cellular communication protocols. The processor 10 may use the communication circuitry 52 to transmit and receive frames or data according to the supported wireless communication protocol. The frames may carry a payload data comprising the time series data measured by the sensors 30. In some embodiments, the processor 10 may use the communication circuitry 52 to transmit the time series data, segment power values, set of scatter points, performance power curve, set of training zones, and/or training instruction to another apparatus, e.g. to the server computer or the wearable device.

In the case of the distributed apparatus or system, a first apparatus such as the server computer may be configured to perform some steps of the method of FIG. 2, such as steps 200, 202, 204, 206, and 210. A second apparatus, such as the wearable device may be configured to perform the steps 212 and 214, for example. The first apparatus may transmit the outputs of one or more steps, such those of step 210 (the set of training zones) to the second apparatus to facilitate subsequent processing of the outputs by the second apparatus. Referring now to FIG. 11, in another example, the server computer may perform steps 1110, 1112, 1114, 1122, and 1124, and the wearable device may perform steps 1116, 1118, and 1120.

The memory 20 may store a computer program product 22 defining computer program instructions for carrying out the method of FIG. 2 or any one of the embodiments thereof. The memory may further store a measurement database 24 comprising the measured the time series data, and/or data associated with and/or derived from the time series data, including e.g. the segment power values, set of scatter points, performance power curve, set of training zones, and/or the training instruction. The memory may further store a user profile 26 storing personal characteristics of the user, e.g. age, weight, etc.

The time series data may include data measured from the user by one or more sensors, such as sensors 30 and 32. The sensors 30 and/or 32 may be configured to measure the time series data from the user. The apparatus may comprise the sensors 30 and/or 32. The sensors may comprise one or more of the following: one or more heart activity sensors, one or more motion sensors, one or more location sensors, one or more barometric sensors, one or more swimming sensors, one or more power sensors, one or more bike sensors, and/or one or more temperature sensors.

The heart activity sensors may be configured to determine heart activity, such as heart rate, heart beat interval (HBI) and/or heart rate variability (HRV), for example. The heart activity sensors include, but are not limited to, a cardiovascular sensor (such as an ECG sensor), an optical heart activity sensor such as a photoplethysmography (PPG) sensor, or a bioimpedance plethysmography. The optical heart activity sensor may detect the heart activity of the user by optical heart rate measurement, which may comprise sending a light beam towards skin of the user and measuring the bounced and/or emitted light from the skin of the user. The light beam may alter when travelling through veins of the user and the alterations may be detected by the optical heart rate activity sensor. An embodiment of an ECG sensor is a wearable sensor device 106, such as a heart rate monitor belt illustrated in FIG. 1, for example. A PPG sensor may be integrated to a wearable device 102, such as a wrist device illustrated in FIG. 1, for example. Further, besides these types of heart activity sensors, other types of biosignal measurement sensors may be embedded into the heart activity sensors. These types include but are not limited to the following: a Laser Doppler-based blood flow sensor, a magnetic blood flow sensor, an electromechanical film (EMFi) pulse sensor, a polarization blood flow sensor. In an embodiment, the heart activity sensor may produce raw sensor data of the heart activity and/or it may process the sensor data into heart activity information, such as heart rate data, for example.

Motion sensors may be configured to measure motion induced by the user to the motion sensors by moving their hands, chest, head, or other body parts to which the motion sensor attached to. The motion sensor may use other motion data, such as location data of the user, to determine motion of the user. In an example embodiment, the motion sensor comprises at least one of the following: an accelerometer, a magnetometer, and a gyroscope. The motion sensor may further comprise sensor fusion software for combining the accelerometer data and gyroscope data so as to provide physical quantities, such as acceleration data, velocity data, or limb trajectory data in a reference coordinate system having orientation defined by a predetermined gyroscope orientation.

Location sensors may utilize a global navigation satellite system (GNSS) or other satellite-based, or radio system-based system for locating the user and measuring various parameters (speed, distance, location, route, altitude, gradient) relating to the movement of the user. Indoor location may be detected via indoor location tracking methods, such as mapping techniques including measuring Earth's magnetic fields or radio frequency signals.

Barometric sensors or altimeters may measure altitude or gradient by measuring atmospheric pressure and matching the measured atmospheric pressure to an altitude.

Swimming sensors may measure swimming specific parameters such as number of strokes or distance, for example.

Power sensors may measure power in various sports. Examples of power sensors include bike power sensors that may be fitted to a bike of the user, and running power sensors, such as footpods.

Bike sensors may be sensors attached to various parts of a (stationary) bike for measuring speed, cadence, or power (output), for example.

The time series data measured by the sensors, or determined by the apparatus on the basis of the time series data, may comprise: heart rate zones, heart rate samples, heart rate variation samples, heart beat interval samples, fat consumption rate, calorie consumption rate, consumed amount of calories, activity zones, activity samples, speed and/or pace samples, power samples, cadence samples, altitude samples, gradient samples, temperature samples, location samples, distance elapsed, time elapsed, pedal index, left-right balance, running index, training load, galvanic skin response samples, fluid balance, skin temperature samples, heading samples and/or bike angles.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

In an embodiment, at least some of the processes described in connection with FIG. 2 may be carried out by a system comprising corresponding means for carrying out at least some of the described processes. Some example means for carrying out the processes may include at least one of the following: detector, processor (including dual-core and multiple-core processors), digital signal processor, controller, receiver, transmitter, encoder, decoder, memory, RAM, ROM, software, firmware, display, user interface, display circuitry, user interface circuitry, user interface software, display software, circuit, and circuitry. In an embodiment, the at least one processor, the memory, and the computer program code form processing means or comprises one or more computer program code portions for carrying out one or more operations according to any one of the embodiments of FIG. 2 or operations thereof.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, other electronic units designed to perform the functions described herein, or a combination thereof. For firmware or software, the implementation can be carried out through modules of at least one chipset (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

Embodiments as described may also be carried out in the form of a computer process defined by a computer program or portions thereof. Embodiments of the methods described in connection with FIG. 2 may be carried out by executing at least one portion of a computer program comprising corresponding instructions. The computer program may be in source code form, object code form, or in some intermediate form, and it may be stored in some sort of carrier, which may be any entity or device capable of carrying the program. For example, the computer program may be stored on a computer program distribution medium 50 readable by a computer or a processor. The computer program medium may be, for example but not limited to, a record medium, computer memory, read-only memory, electrical carrier signal, telecommunications signal, and software distribution package, for example. The computer program medium may be a non-transitory medium. Coding of software for carrying out the embodiments as shown and described is well within the scope of a person of ordinary skill in the art.

Even though the invention has been described with reference to one or more embodiments according to the accompanying drawings, it is clear that the invention is not restricted thereto but may be modified in several ways within the scope of the appended claims. All words and expressions should be interpreted broadly, and they are intended to illustrate, not to restrict, the embodiments. It will be obvious to a person skilled in the art that, as technology advances, the inventive concept may be implemented in various ways.

**Claims**

1. An apparatus comprising means for:

   acquiring (200) time series data that represents power output of athletic performances of a user (100);
   extracting (202) a plurality of segments from the time series data, wherein the plurality of segments comprise a first segment having a first duration and a second segment having a second duration, the second duration being different from the first duration;
   determining (204) segment power values on the basis of the time series data of the extracted plurality of segments;
   forming (206) a set of scatter points describing the segment power values as a function of respective durations of the extracted plurality of segments, and fitting (208) a performance power curve for the set of scatter points;
   determining (210) a set of training zones on the basis of the performance power curve;
   generating (212) a training instruction on the basis of the set of training zones; and
   causing (214) a user interface to output the training instruction to the user.

2. The apparatus of any preceding claim, wherein the means are configured to compute (216) segment average values from the time series data of the extracted plurality of segments to determine the segment power values.

3. The apparatus of any preceding claim, wherein the time series data comprises velocity data and/or heart rate data, and wherein the means are configured to convert (218) the velocity data and/or the heart rate data to power output data, and to determine the segment power values on the basis of the power output data.

4. The apparatus of any preceding claim, wherein the means are configured to:

   obtain (500, 502) an input parameter and a training target;
   select (504), on the basis of the training target, a training zone from the set of training zones;
   determine (506) a power output target on the basis of the input parameter and the selected training zone; and
   cause (508) the user interface to output the power output target.

5. The apparatus of claim 4, wherein the means are configured to convert (600) the power output target to a heart rate target and/or a velocity target, and wherein the means are configured to cause (604) the user interface to output the heart rate target and/or the velocity target as the power output target.

6. The apparatus of claim 5, wherein the means are configured to convert the power output target to a heart rate target and to perform (602) a drift correction on the heart rate target, and to cause the user interface to output the corrected heart rate target as the power output target.

7. The apparatus of any preceding claim 4-6, wherein the input parameter is a duration parameter or a distance parameter.

8. The apparatus of any preceding claim, wherein the first duration is smaller than a crossover time representing a time for maintaining maximal aerobic speed, and the second duration is greater than the crossover time.

9. The apparatus of any preceding claim, wherein the means are configured to:

divide (700) the set of scatter points to a first set of scatter points (800) and a second set of scatter points (802), wherein durations of the first set of scatter points are smaller than or equal to a cut-off time, and durations of the second set of scatter points are greater than or equal to the cut-off time; and

perform (702) the fitting for the first set of scatter points to obtain a first piece (804) of the performance power curve, and perform (704) the fitting for the second set of scatter points to obtain a second piece (806) of the performance power curve, and form (706) the performance power curve on the basis of the first piece of the performance power curve and the second piece of the performance power curve.

10. The apparatus of claim 9, wherein the performance power curve is a piecewise linear function.

11. The apparatus of any preceding claim, wherein the means are configured to determine a training zone (402, 404, 406) of the set of training zones by multiplying the performance power curve (400) by a constant multiplier.

12. The apparatus of any preceding claim, wherein the means are configured to:

obtain (900) training plan data (1000) associated with the time series data (1002);

identify (902), on the basis of the training plan data, one or more time periods of the time series data that have training intensities greater than an inclusion threshold; and

extract (904) the plurality of segments from the time series data of the identified one or more time periods.

13. The apparatus of claim 12, wherein the one or more time periods comprise one or more training interval periods (1012, 1016), and wherein the means are configured to extract one or more of the plurality of segments as the one or more training interval periods.

14. A method comprising:

acquiring (200) time series data that represents power output of athletic performances of a user;

extracting (202) a plurality of segments from the time series data, wherein the plurality of segments comprise a first segment having a first duration and a second segment having a second duration, the second duration being different from the first duration;

determining (204) segment power values on the basis of the time series data of the extracted plurality of segments;

forming (206) a set of scatter points describing the segment power values as a function of respective durations of the extracted plurality of segments, and fitting (208) a performance power curve for the set of scatter points;

determining (210) a set of training zones on the basis of the performance power curve;

generating (212) a training instruction on the basis of the set of training zones; and

causing (214) a user interface to output the training instruction to the user.

15. A computer program product readable by a computer and comprising program instructions which, when executed by the computer, cause the computer to carry out a computer process comprising:

acquiring (200) time series data that represents power output of athletic performances of a user;

extracting (202) a plurality of segments from the time series data, wherein the plurality of segments comprise a first segment having a first duration and a second segment having a second duration, the second duration being different from the first duration;

determining (204) segment power values on the basis of the time series data of the extracted plurality of segments;

forming (206) a set of scatter points describing the segment power values as a function of respective durations of the extracted plurality of segments, and fitting (208) a performance power curve for the set of scatter points;

determining (210) a set of training zones on the basis of the performance power curve;

generating (212) a training instruction on the basis of the set of training zones; and

causing (214) a user interface to output the training instruction to the user.

FIG. 1

START

200 ACQUIRE TIME SERIES DATA

202 EXTRACT SEGMENTS

218 CONVERT TO POWER OUTPUT DATA

204 DETERMINE SEGMENT POWER VALUES

216 COMPUTE SEGMENT AVERAGE VALUES

206 FORM SCATTER POINTS

208 FIT PERFORMANCE POWER CURVE

210 DETERMINE TRAINING ZONES

212 GENERATE TRAINING INSTRUCTION

214 OUTPUT TRAINING INSTRUCTION

END

FIG. 2

FIG. 3

FIG. 4

500 OBTAIN INPUT PARAMETER

502 OBTAIN TRAINING TARGET

504 SELECT TRAINING ZONE

506 DETERMINE POWER OUTPUT TARGET

508 OUTPUT POWER OUTPUT TARGET

FIG. 5

600 CONVERT TARGET

602 PERFORM DRIFT CORRECTION

604 OUTPUT TARGET

FIG. 6

700 DIVIDE SCATTER
POINTS TO FIRST SET AND
SECOND SET

702 PERFORM FITTING FOR
FIRST SET

704 PERFORM FITTING FOR
SECOND SET

706 FORM PERFORMANCE
POWER CURVE

FIG. 7

FIG. 8

```
┌─────────────────────────┐
│  900 OBTAIN TRAINING    │
│      PLAN DATA          │
└─────────────────────────┘
            │
            ▼
┌─────────────────────────┐
│  902 IDENTIFY TIME      │
│      PERIODS            │
└─────────────────────────┘
            │
            ▼
┌─────────────────────────┐
│  904 EXTRACT SEGMENTS   │
│    FROM TIME PERIODS    │
└─────────────────────────┘
```

# FIG. 9

FIG. 10

START

1110 SELECT TRAINING PROGRAM

1112 DETERMINE TRAINING TARGET

1114 DETERMINE INPUT PARAMETER

1116 START TRAINING SESSION

1118 OUTPUT TRAINING GUIDANCE

1120 ACQUIRE SENSOR DATA

YES ◁— 1122 KEY EXERCISE? —▷ NO

1124 UPDATE PERFORMANCE PROFILE CURVE

END

FIG. 11

**30 SENSOR(S)**

**52 COMMUNICATION CIRCUITRY**

**10 PROCESSOR**

**14 MEASUREMENT SIGNAL PROCESSING**

**16 TRAINING CONTROLLER**

**20 MEMORY**

**22 SOFTWARE**

**24 MEASUREMENT DATABASE**

**26 USER PROFILE**

**50 MEDIUM**

**40 USER INTERFACE**

**32 SENSOR(S)**

FIG. 12

Europäisches
Patentamt

European
Patent Office

Office européen
des brevets

# EUROPEAN SEARCH REPORT

Application Number

EP 22 16 8347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/263439 A1 (ACKLAND JONATHAN EDWARD BELL [NZ]) 15 September 2016 (2016-09-15) | 1-5,7, 9-15 | INV. G16H20/30 |
| Y | * paragraphs [0208], [0167], [0676], [0120]; figure 3 * | 6,8 | |
| A | D. C. CLARKE ET AL: "Rationale and resources for teaching the mathematical modeling of athletic training and performance", ADVANCES IN PHYSIOLOGY EDUCATION, vol. 37, no. 2, 1 June 2013 (2013-06-01), pages 134-152, XP055380347, US ISSN: 1043-4046, DOI: 10.1152/advan.00078.2011 * page 134, column 1, paragraph 1 * * page 138, column 2; figure 3 * * page 139, column 2 * | 1-15 | |
| Y | US 2022/032125 A1 (AULET THOMAS [US] ET AL) 3 February 2022 (2022-02-03) * paragraphs [0002], [0003] * | 6 | TECHNICAL FIELDS SEARCHED (IPC) G16H |
| Y | CN 106 202 953 B (BEIJING XINLIANG SCIENCE AND TECH CO LTD) 2 July 2019 (2019-07-02) * paragraph [0146] * | 8 | |
| A | JP 2013 022090 A (UNIV TOHOKU) 4 February 2013 (2013-02-04) * the whole document * | 1-15 | |
| A | EP 2 682 052 A2 (ADIDAS AG [DE]) 8 January 2014 (2014-01-08) * the whole document * | 1-15 | |

The present search report has been drawn up for all claims

| Place of search | Date of completion of the search | Examiner |
|---|---|---|
| Munich | 9 September 2022 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 8347

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2022

| Patent document cited in search report | | Publication date | Patent family member(s) | | Publication date |
|---|---|---|---|---|---|
| US 2016263439 | A1 | 15-09-2016 | AU | 2014347366 A1 | 23-06-2016 |
| | | | AU | 2019284143 A1 | 30-01-2020 |
| | | | EP | 3066592 A1 | 14-09-2016 |
| | | | US | 2016263439 A1 | 15-09-2016 |
| | | | US | 2019388733 A1 | 26-12-2019 |
| | | | US | 2020368580 A1 | 26-11-2020 |
| | | | WO | 2015069124 A1 | 14-05-2015 |
| US 2022032125 | A1 | 03-02-2022 | US | 2022032120 A1 | 03-02-2022 |
| | | | US | 2022032125 A1 | 03-02-2022 |
| | | | WO | 2022026929 A1 | 03-02-2022 |
| CN 106202953 | B | 02-07-2019 | NONE | | |
| JP 2013022090 | A | 04-02-2013 | JP | 5888582 B2 | 22-03-2016 |
| | | | JP | 2013022090 A | 04-02-2013 |
| EP 2682052 | A2 | 08-01-2014 | CN | 103520897 A | 22-01-2014 |
| | | | EP | 2682052 A2 | 08-01-2014 |
| | | | JP | 6306833 B2 | 04-04-2018 |
| | | | JP | 2014014683 A | 30-01-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MULLIGAN et al.** A minimal power model for human running performance. *PLoS ONE,* 2018, vol. 13 (11), e0206645
- **EMIG ; PELTONEN.** Human running performance from real-world big data. *Nat Commun,* 2020, vol. 11, 4936
- **STIRLING, JAMES ROBERT et al.** A Model of Heart Rate Kinetics in Response to Exercise. *Journal of Nonlinear Mathematical Physics,* October 2008, vol. 15 (3), 426-436